(19) Europäisches Patentamt
European Patent Office
Office européen des brevets

(11) Veröffentlichungsnummer: **0 282 811**
**A1**

# (12) EUROPÄISCHE PATENTANMELDUNG

(21) Anmeldenummer: **88103123.1**

(22) Anmeldetag: **02.03.88**

(51) Int. Cl.⁴: **C07D 403/04** , C07D 401/04 , A01N 43/56 , //C07D231/40

(30) Priorität: **11.03.87 DE 3707686**

(43) Veröffentlichungstag der Anmeldung: **21.09.88 Patentblatt 88/38**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnöckel 49**
**D-5600 Wuppertal 11(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**

## (54) 4-Cyano-1-aryl-pyrazole.

(57) 4-Cyano-1-arylpyrazole der allgemeinen Formel I

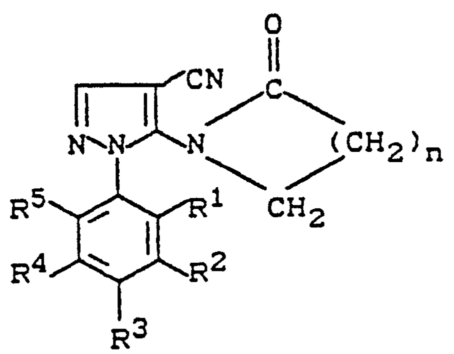

(I)

in welcher
$R^1$, $R^2$, $R^3$, $R^4$ und $R^5$ unabhängig voneinander jeweils für Wasserstoff, Halogen, Cyano, Nitro, Alkyl, Alkoxy, Alkylsulfonyl, Alkoxycarbonyl oder für einen Rest $-(X)_m-R^6$ stehen, wobei
X für Sauerstoff, Schwefel, Sulfinyl oder Sulfonyl steht,
m für eine Zahl 0 oder 1 steht,

EP 0 282 811 A1

$R^6$ für Halogenalkyl steht und

n für eine Zahl 1, 2 oder 3 steht,

mit der Maßgabe, daß mindestens einer der Reste $R^1$, $R^2$, $R^3$, $R^4$ oder $R^5$ für einen Rest $-(X)_m-R^6$ steht, wobei jedoch $R^3$ nicht für Trifluormethyl steht,

ein Verfahren zu deren Herstellung und ihre Verwendung als Herbizide.

# 4-Cyano-1-aryl-pyrazole

Die Erfindung betrifft neue 4-Cyano-1-aryl-pyrazole, ein Verfahren zu ihrer Herstellung und ihre Verwendung als Herbizide.

Es ist bereits bekannt, daß bestimmte 4-Cyano-1-aryl-pyrazole, wie beispielsweise das 4-Cyano-5-propionamido-1-(2,4,6-trichlorphenyl)-pyrazol herbizide Eigenschaften besitzen (vergl. DE-OS 32 26 513).

Die herbizide Wirksamkeit dieser vorbekannten Verbindungen gegenüber schwer bekämpfbaren Problemunkräutern ist jedoch ebenso wie ihre Verträglichkeit gegenüber wichtigen Kulturpflanzen nicht in allen Anwendungsbereichen völlig zufriedenstellend.

Es wurden neue 4-Cyano-1-aryl-pyrazole der allgemeinen Formel (I),

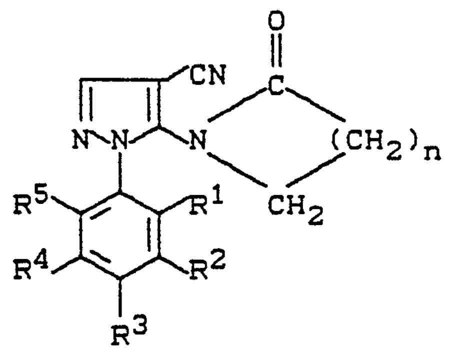

(I)

in welcher

$R^1$, $R^2$, $R^3$, $R^4$ und $R^5$ unabhängig voneinander jeweils für Wasserstoff, Halogen, Cyano, Nitro, Alkyl, Alkoxy, Alkylsulfonyl, Alkoxycarbonyl oder für einen Rest $-(X)_m-R^6$ stehen, wobei

X für Sauerstoff, Schwefel, Sulfinyl oder Sulfonyl steht,

m für eine Zahl 0 oder 1 steht,

$R^6$ für Halogenalkyl steht und

n für eine Zahl 1, 2 oder 3 steht,

mit der Maßgabe, daß mindestens einer der Reste $R^1$, $R^2$, $R^3$, $R^4$ oder $R^5$ für einen Rest $-(X)_m-R^6$ steht, wobei je doch $R^3$ nicht für Trifluormethyl steht,

gefunden.

Weiterhin wurde gefunden, daß man die neuen 4-Cyano-1-aryl-pyrazole der allgemeinen Formel (I),

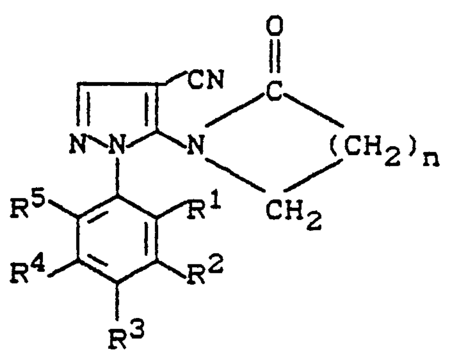

(I)

in welcher

$R^1$, $R^2$, $R^3$, $R^4$, $R^5$ und n die oben angegebene Bedeutung haben,

erhält, wenn man 5-Amino-4-cyano-1-aryl-pyrazole der Formel (II),

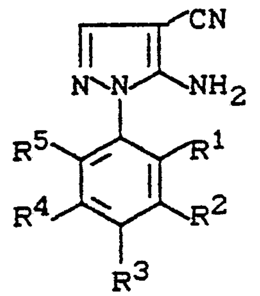

(II)

in welcher

$R^1$, $R^2$, $R^3$, $R^4$ und $R^5$ die oben angegebene Bedeutung haben,

mit Chloralkanoylchloriden der Formel (III),

$Cl-\overset{O}{\overset{\|}{C}}-(CH_2)_n-CH_2-Cl$ (III)

in welcher

n die oben angegebene Bedeutung hat,

gegebenenfalls in Gegenwart eines Verdünnungsmittels und gegebenenfalls in Gegenwart eines Reaktionshilfsmittels umsetzt und die dabei auftretenden Zwischenprodukte der Formel (IV),

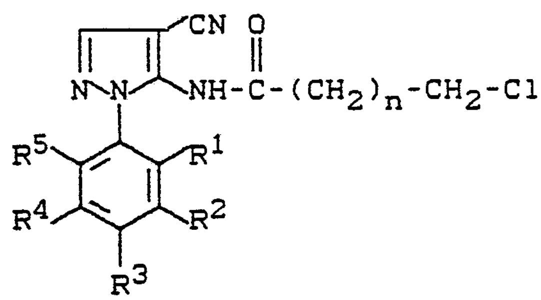

(IV)

in welcher

$R^1$, $R^2$, $R^3$, $R^4$, $R^5$ und n die oben angegebene Bedeutung haben,

entweder direkt in einem Reaktionsschritt ("Eintopfverfahren") oder in einer separaten 2. Reaktionsstufe cyclisiert.

Schließlich wurde gefunden, daß die neuen 4-Cyano-1-aryl-pyrazole der allgemeinen Formel (I) herbizide Eigenschaften besitzen.

Überraschenderweise zeigen die erfindungsgemäßen 4-Cyano-1-aryl-pyrazole der allgemeinen Formel (I) neben einer erheblich verbesserten herbiziden Wirksamkeit gegenüber schwer bekämpfbaren Problemunkräutern gleichzeitig eine deutlich verbesserte Kulturpflanzenverträglichkeit im Vergleich zu den aus dem Stand der Technik bekannten 4-Cyano-1-aryl-pyrazolen, wie beispielsweise das 4-Cyano-5-propionamido-1-(2,4,6-trichlorphenyl)-pyrazol, welches chemisch und wirkungsmäßig naheliegende Verbindungen sind.

Die erfindungsgemäßen 4-Cyano-1-aryl-pyrazole sind durch die Formel (I) allgemein definiert. Bevorzugt sind Verbindungen der Formel (I), bei welchen

$R^1$, $R^2$, $R^3$, $R^4$ und $R^5$ unabhängig voneinander für Wasserstoff, Fluor, Chlor, Brom, Iod, Cyano, Nitro, jeweils geradkettiges oder verzweigtes Alkyl, Alkoxy, Alkylsulfonyl oder Alkoxycarbonyl mit 1 bis 4 Kohlenstoffatomen in den jeweiligen Alkylteilen oder für einen Rest $-(X)_m-R^6$ stehen, wobei

X für Sauerstoff, Schwefel, Sulfinyl oder Sulfonyl steht,

m für 0 oder 1 steht und

$R^6$ für geradkettiges oder verzweigtes Halogenalkyl mit 1 bis 4 Kohlenstoffatomen und 1 bis 9 gleichen oder verschiedenen Halogenatomen steht, und

n für eine Zahl 1, 2 oder 3 steht,

mit der Maßgabe, daß mindestens einer der Reste $R^1$, $R^2$, $R^3$, $R^4$ oder $R^5$ für einen Rest $-(X)_m-R^6$ steht, wobei jedoch $R^3$ nicht für Trifluormethyl steht.

Besonders bevorzugt sind Verbindungen der Formel (I), bei welchen

$R^1$, $R^2$, $R^3$, $R^4$ und $R^5$ unabhängig voneinander für Wasserstoff, Fluor, Chlor, Brom, Cyano, Nitro, Methyl, Ethyl, n-oder i-Propyl, Methoxy, Ethoxy, Methylsulfonyl, Methoxycarbonyl, Ethoxycarbonyl oder für einen

Rest $-(X)_m-R^6$ stehen, wobei
X für Sauerstoff, Schwefel, Sulfinyl oder Sulfonyl steht,
m für 0 oder 1 steht und
$R^6$ für Trifluormethyl, Trichlormethyl, Dichlorfluormethyl, Difluorchlormethyl Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl steht und
n für eine Zahl 1, 2 oder 3 steht,
mit der Maßgabe, daß mindestens einer der Reste $R^1$, $R^2$, $R^3$, $R^4$ oder $R^5$ für einen Rest $-(X)_m-R^6$ steht, wobei jedoch $R^3$ nicht für Trifluormethyl steht.

Im einzelnen seien außer den bei den Herstellungsbeispielen genannten Verbindungen die folgenden 4-Cyano-1-aryl-pyrazole der allgemeinen Formel (I) genannt:

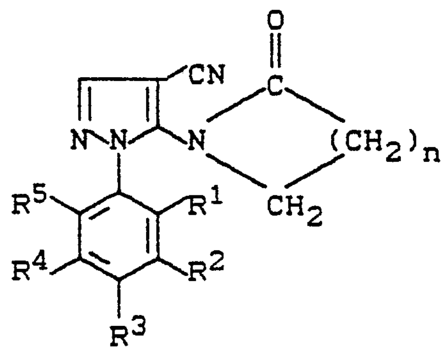

(I)

| $R^1$ | $R^2$ | $R^3$ | $R^4$ | $R^5$ | n |
|---|---|---|---|---|---|
| Cl | H | $SO_2CF_3$ | H | Cl | 1 |
| Cl | H | $SO_2CF_3$ | H | Cl | 3 |
| Cl | H | $SCF_3$ | H | H | 1 |
| Cl | H | $SCF_3$ | H | H | 2 |
| Cl | H | $OCF_3$ | H | Cl | 1 |
| Cl | Cl | $OCF_3$ | H | Cl | 3 |
| Cl | H | $OCF_3$ | H | Cl | 3 |
| Cl | Cl | $SCF_3$ | H | Cl | 1 |
| Cl | Cl | $SCF_3$ | H | Cl | 2 |
| Cl | Cl | $OCF_3$ | H | Cl | 1 |
| Cl | Cl | $OCF_3$ | H | Cl | 2 |

Verwendet man beispielsweise 4-Cyano-5-amino-1-(2-chlor-4-trifluormethoxyphenyl)-pyrazol und 3-Chlorpropionylchlorid als Ausgangsstoffe, so läßt sich der Reaktionsablauf des erfindungsgemäßen Verfahrens durch das folgende Formelschema darstellen:

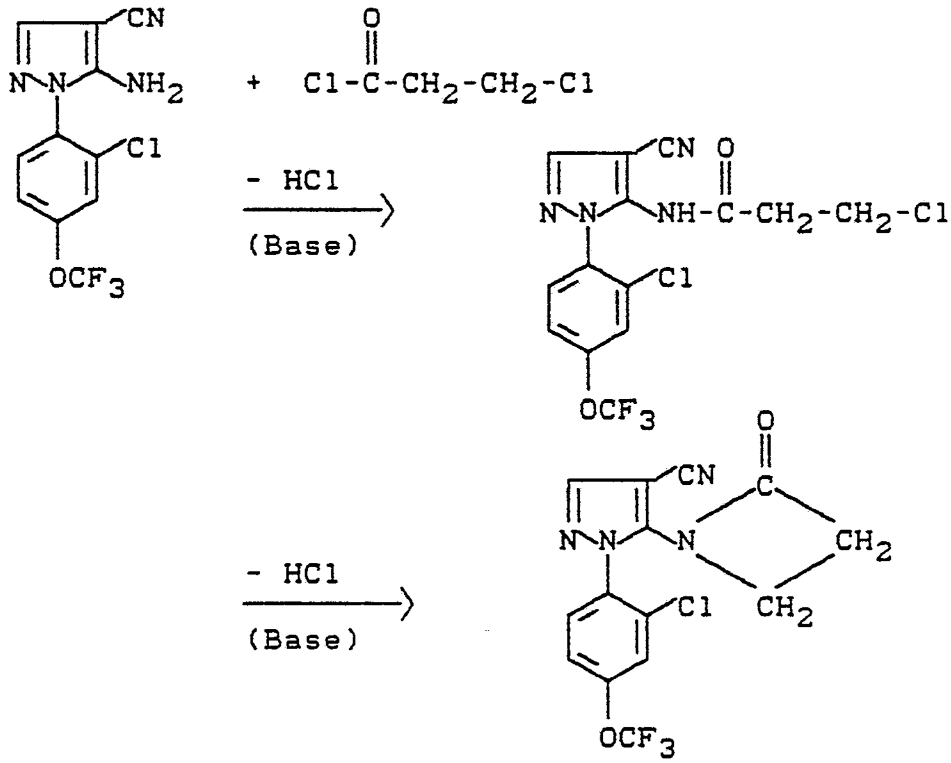


Die zur Durchführung des erfindungsgemäßen Verfahrens als Ausgangsstoffe benötigten 5-Amino-4-cyano-1-aryl-pyrazole sind durch die Formel (II) allgemein definiert. In dieser Formel (II) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-4-cyano-1-aryl-pyrazole der Formel (II) sind bekannt oder herstellbar in Analogie zu bekannten Verfahren (vergl. EP 138 149 oder EP 34 945).

Die zur Durchführung des erfindungsgemäßen Verfahrens weiterhin als Ausgangsstoffe benötigten Chloralkanoylchloride sind durch die Formel (III) allgemein definiert. In dieser Formel (III) steht der Index n vorzugsweise für diejenigen Zahlen, die bereits im Zusammenhang mit der Beschreibung der erfindungsgemäßen Stoffe der Formel (I) als bevorzugt für diesen Index genannt wurden.

Die Chloralkanoylchloride der Formel (III) sind allgemein bekannte Verbindungen der organischen Chemie.

Als Verdünnungsmittel zur Durchführung des erfindungsgemäßen Verfahrens kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl-oder -diethylether; Ketone wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; oder Sulfoxide, wie Dimethylsulfoxid.

Das erfindungsgemäße Verfahren kann gegebenenfalls in Gegenwart eines geeigneten Säurebindemittels durchgeführt werden.

Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens setzt man pro Mol an 5-Amino-4-cyano-1-arylpyrazol der Formel (II) im allgemeinen 1.0 bis 5.0 Mol, vorzugsweise 1.0 bis 2.5 Mol an Chloralkanoylchlorid der Formel (III) und 1.0 bis 5.0 Mol, vorzugsweise 1.0 bis 2.5 Mol an Säurebindemittel ein. Die Reaktionsdurchführung, Aufarbeitung und Isolierung der Reaktionsprodukte erfolgt sowohl bei der einstufigen als auch bei der zweistufigen Reaktionsvariante in Analogie zu bekannten Verfahren mit Hilfe allgemein üblicher Methoden (vergl. DE-OS 33 25 488, EP 205 021 sowie die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono-und dikotyler Unkräuter in monokotylen Kulturen wie beispielsweise Hafer oder Weizen einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formu lierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmoril lonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit 4-Ethylamino-2-t-butylamino-6-methylthio-s-triazin; 4-Amino-6-t-butyl-3-ethylthio-1,2,4-triazin-5(4H)-on; 2-Chlor-N-{[(4-methoxy-6-methyl-1,3,5-triazin-2-yl)-amino]-carbonyl}-benzolsulfonamid; N,N-Dimethyl-N'-(4-isopropylphenyl)-harnstoff; N,N-Dimethyl-N'-(3-chlor-4-methylphenyl)-harnstoff; N-Methyl-2-(1,3-benzthiazol-2-yloxy)-acetanilid; N-(1-Ethylpropyl)-3,4-dimethyl-2,6-dinitroanilin; N,N-Diisoproyl-S-(2,3,3-trichlorallyl)-thiolcarbamat; 2-[4-(2,4-Dichlorphenoxy)-phenoxy]-propionsäure-methylester; 2-[4-(3,5-Dichlorpyrid-2-yloxy)-phenoxy]-propionsäure-(trimethylsilylmethylester); 2,4-Dichlorphenoxyessigsäure; 2,4-Dichlorphenoxypropionsäure; (4-Chlor-2-methylphenoxy)-propionsäure; (2-Me thyl-4-chlorphenoxy)-essigsäure; Methyl-5-(2,4-dichlorphenoxy)-2-nitrobenzoat; 3,5-Diiod-4-hydroxybenzonitril; 3,5-Dibrom-4-hydroxy-benzonitril; [(4-Amino-3,5-dichlor-6-fluor-2-pyridinyl)-oxy]-essigsäure; bzw. -1-methylheptylester oder 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

(zweistufige Reaktionsführung)

a)

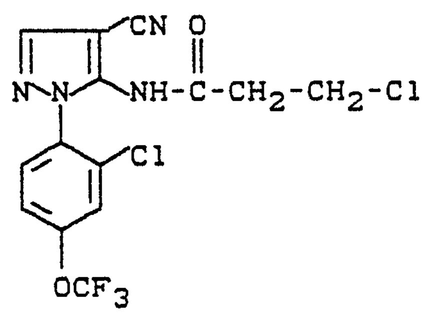

Zu 7,6 g (0.025 Mol) 1-(2-Chlor-4-trifluormethoxyphenyl)-4-cyan-5-amino-pyrazol in 100 ml Acetonitril tropft man bei Raumtemperatur unter Rühren 15,8 g (0.125 Mol) 3-Chlorpropionsäurechlorid in 20 ml Acetonitril. Es wird 5 Tage bei Raumtemperatur gerührt, das Lösungsmittel und überschüssiges Säurechlorid im Vakuum entfernt und der schmierige Rückstand durch Anreiben mit Petrolether zur Kristallisation gebracht.

Nach einmaligem Umkristallisieren aus Toluol erhält man 5,8 g (59 % der Theorie) an 5-(3-Chlorpropionamido)-1-(2-chlor-4-trifluormethoxy-phenyl)-4-cyano-pyrazol vom Schmelzpunkt 125 °C.

b)

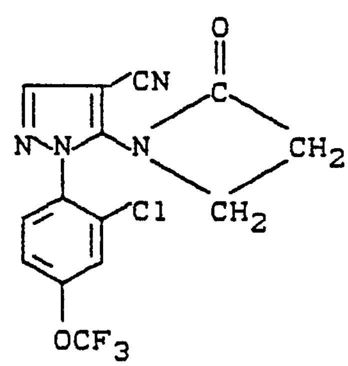

3,9 g (0.01 Mol) 5-(3-Chlorpropionamido)-1-(2-chlor-4-trifluormethoxy-phenyl)-4-cyano-pyrazol und 3,5 g (0.035 Mol) Kaliumcarbonat in 60 ml Aceton werden 2 Stunden lang unter Rückfluß erhitzt. Zur Aufarbeitung wird die erkaltete Reaktionsmischung filtriert, das Lösungsmittel abdestilliert und der Rückstand chromatographisch gereinigt (Kieselgel; Chloroform/Aceton 9 : 1).

Man erhält 0,8 g (22 % der Theorie) an 1-(2-Chlor-4-trifluormethoxyphenyl)-4-cyano-5-(2-oxo-azetidin-1-yl)-pyrazol von Schmelzpunkt 117 °C - 119 °C.

Beispiel 2

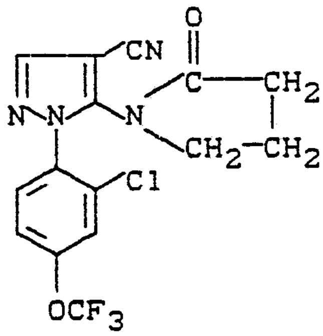

("Eintopfverfahren")

Zu 7,6 g (0.025 Mol) 1-(2-Chlor-4-trifluormethoxyphenyl)-4-cyano-5-amino-pyrazol in 100 ml Acetonitril tropft man bei Raumtemperatur unter Rühren 7,1 g (0.05 Mol) 4-Chlorbutyrylchlorid in 20 ml Acetonitril, erwärmt nach beendeter Zugabe 16 Stunden lang auf Rückflußtemperatur, kühlt wieder ab auf Raumtemperatur und entfernt das Lösungsmittel im Vakuum. Der Rückstand wird in 50 ml Aceton aufgenommen, mit 8 g (0.058 Mol) Kaliumcarbonat versetzt, 30 Minuten auf Rückflüßtemperatur erwärmt, filtriert, im Vakuum eingeengt und chromatographisch gereinigt (Kieselgel; Chloroform/Aceton 9 : 1).

Man erhält 3,9 g (53 % der Theorie) an 1-(2-Chlor-4-trifluormethoxy-phenyl)-4-cyano-5-(2-oxopyrrolidin-1-yl)-pyrazol vom Schmelzpunkt 84 °C.

Herstellung der Ausgangsverbindung

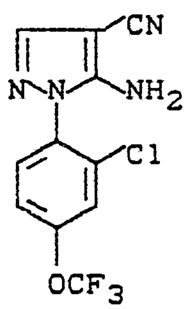

3,08 g (0.025 Mol) Ethoxymethylenmalonsäuredinitril und 5,7 g (0.025 Mol) 2-Chlor-4-trifluormethoxyphenylhydrazin in 50 ml Ethylenglykolmonoethylether werden 3 Stunden unter Rückfluß erhitzt, nach dem Abkühlen auf Wasser gegossen, der kristalline Niederschlag abgesaugt, mit Petrolether verrührt, gekühlt und abermals abgesaugt.

Man erhält 5,3 g (73,6 % der Theorie) an 5-Amino-4-cyano-1-(2-chlor-4-trifluormethoxyphenyl)-pyrazol vom Schmelzpunkt 115 °C.

In entsprechender Weise und gemäß den allgemeinen Angaben zur Herstellung erhält man die folgenden 4-Cyano-1-aryl-pyrazole der allgemeinen Formel (I):

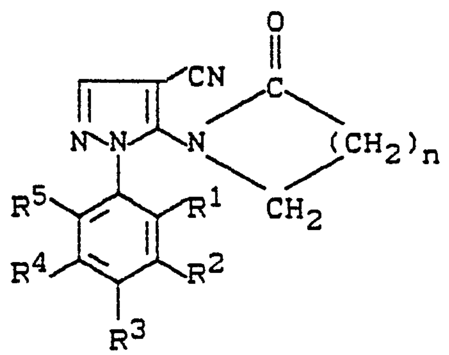

(I)

| Beisp.-Nr. | $R^1$ | $R^2$ | $R^3$ | $R^4$ | $R^5$ | n | Schmelzpunkt /° C |
|---|---|---|---|---|---|---|---|
| 3 | Cl | H | $SCF_3$ | H | Cl | 1 | 137 - 142 |
| 4 | Cl | H | $SCF_3$ | H | Cl | 2 | 129 - 131 |
| 5 | Cl | H | $SO_2CF_3$ | H | Cl | 2 | 142 - 144 |
| 6 | Cl | H | $OCF_3$ | H | Cl | 2 | 120 - 123 |

Anwendungsbeispiele:

In den folgenden Anwendungsbeispielen wurde die nachstehend aufgeführte Verbindung als Vergleichssubstanz eingesetzt:

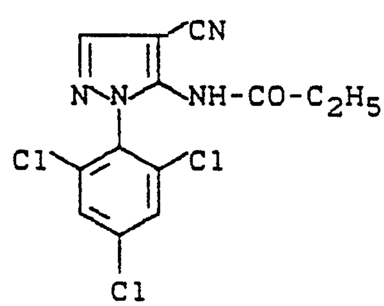

(A)

4-Cyano-5-propionamido-1-(2,4,6-trichlorphenyl)-pyrazol (bekannt aus DE-OS 32 26 513).

Beispiel A

Pre-emergence-Test Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtseil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge

des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigt beispielsweise die Verbindung gemäß Herstellungsbeispiel (1) eine deutliche Überlegenheit in der Wirksamkeit ebenso wie in der Nutzpflanzenselektivität gegenüber dem Stand der Technik.

Beispiel B

Post-emergence-Test Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigt beispielsweise die Verbindung gemäß Herstellungsbeispiel 1 eine deutliche Überlegenheit in der Wirksamkeit ebenso wie in der Nutzpflanzenselektivität gegenüber dem Stand der Technik.

**Ansprüche**

1. 4-Cyano-1-arylpyrazole der allgemeinen Formel (I)

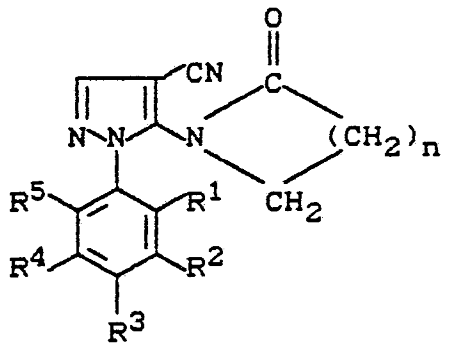

(I)

in welcher

$R^1$, $R^2$, $R^3$, $R^4$ und $R^5$ unabhängig voneinander jeweils für Wasserstoff, Halogen, Cyano, Nitro, Alkyl, Alkoxy, Alkylsulfonyl, Alkoxycarbonyl oder für einen Rest $-(X)_m-R^6$ stehen, wobei

X für Sauerstoff, Schwefel, Sulfinyl oder Sulfonyl steht,

m für eine Zahl 0 oder 1 steht,

$R^6$ für Halogenalkyl steht und

n für eine Zahl 1, 2 oder 3 steht,

mit der Maßgabe, daß mindestens einer der Reste $R^1$, $R^2$, $R^3$, $R^4$ oder $R^5$ für einen Rest $-(X)_m-R^6$ steht, wobei jedoch $R^3$ nicht für Trifluormethyl steht.

2. Verbindungen der allgemeinen Formel (I), gemäß Anspruch 1, bei welchen
$R^1$, $R^2$, $R^3$, $R^4$ und $R^5$ unabhängig voneinander für Wasserstoff, Fluor, Chlor, Brom, Iod, Cyano, Nitro, jeweils geradkettiges oder verzweigtes Alkyl, Alkoxy, Alkylsulfonyl oder Alkoxycarbonyl mit 1 bis 4 Kohlenstoffatomen in den jeweiligen Alkylteilen oder für einen Rest $-(X)_m-R^6$ stehen, wobei
X für Sauerstoff, Schwefel, Sulfinyl oder Sulfonyl steht,
m für 0 oder 1 steht und
$R^6$ für geradkettiges oder verzweigtes Halogenalkyl mit 1 bis 4 Kohlenstoffatomen und 1 bis 9 gleichen oder verschiedenen Halogenatomen steht, und
n für eine Zahl 1, 2 oder 3 steht,
mit der Maßgabe, daß mindestens einer der Reste $R^1$, $R^2$, $R^3$, $R^4$ oder $R^5$ für einen Rest $-(X)_m-R^6$ steht, wobei jedoch $R^3$ nicht für Trifluormethyl steht.

3. Verbindungen der allgemeinen Formel (I) gemäß Anspruch 1, bei welchen
$R^1$, $R^2$, $R^3$, $R^4$ und $R^5$ unabhängig voneinander für Wasserstoff, Fluor, Chlor, Brom, Cyano, Nitro, Methyl, Ethyl, n-oder i-Propyl, Methoxy, Ethoxy, Methylsulfonyl, Methoxycarbonyl, Ethoxycarbonyl oder für einen Rest $-(X)_m-R^6$ stehen, wobei
X für Sauerstoff, Schwefel, Sulfinyl oder Sulfonyl steht,
m für 0 oder 1 steht und
$R^6$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl stehen und
n für eine Zahl 1, 2 oder 3 steht,
mit der Maßgabe, daß mindestens einer der Reste $R^1$, $R^2$, $R^3$, $R^4$ oder $R^5$ für einen Rst $-(X)_m-R^6$ steht, wobei jedoch $R^3$ nicht für Trifluormethyl steht.

4. Verfahren zur Herstellung von 4-Cyano-1-aryl-pyrazolen der allgemeinen Formel (I),

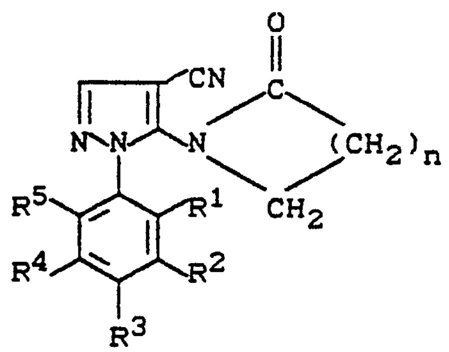

(I)

in welcher
$R^1$, $R^2$, $R^3$, $R^4$ und $R^5$ unabhängig voneinander jeweils für Wasserstoff, Halogen, Cyano, Nitro, Alkyl, Alkoxy, Alkylsulfonyl, Alkoxycarbonyl oder für einen Rest $-(X)_m-R^6$ stehen, wobei
X für Sauerstoff, Schwefel, Sulfinyl oder Sulfonyl steht,
m für eine Zahl 0 oder 1 steht,
$R^6$ für Halogenalkyl steht und
n für eine Zahl 1, 2 oder 3 steht,
mit der Maßgabe, daß mindestens einer der Reste $R^1$, $R^2$, $R^3$, $R^4$ oder $R^5$ für einen Rest $-(X)_m-R^6$ steht, wobei jedoch $R^3$ nicht für Trifluormethyl steht,
dadurch gekennzeichnet, daß man 5-Amino-4-cyano-1-aryl-pyrazole der allgemeinen Formel (II),

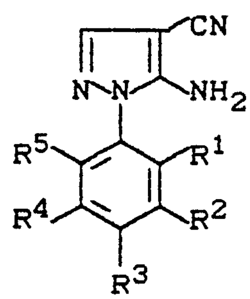

(II)

in welcher
$R^1$, $R^2$, $R^3$, $R^4$ und $R^5$ die oben angegebene Bedeutung haben,
mit Chloralkanoylchloriden der allgemeinen Formel (III),

$$Cl-\overset{\overset{O}{\|}}{C}-(CH_2)_n-CH_2-Cl \qquad (III)$$

in welcher
n die oben angegebene Bedeutung hat,
gegebenenfalls in Gegenwart eines Verdünnungsmittels und gegebenenfalls in Gegenwart eines Reaktionshilfsmittels umsetzt und die dabei auftretenden Zwischenprodukte der allgemeinen Formel (IV),

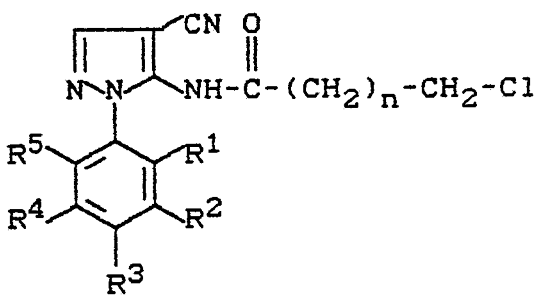

(IV)

in welcher
$R^1$, $R^2$, $R^3$, $R^4$, $R^5$ und n die oben angegebene Bedeutung haben,
entweder direkt in einem Reaktionsschritt ("Eintopfverfahren") oder in einer separaten 2. Reaktionsstufe cyclisiert.

5. Herbizide Mittel, gekennzeichnet durch einen Gehalt an mindestens einem 4-Cyano-5-arylpyrazol der allgemeinen Formel (I) gemäß den Ansprüchen 1 bis 4.

6. Verfahren zur Bekämpfung von Unkräutern, dadurch gekennzeichnet, daß man 4-Cyano-5-arylpyrazole der allgemeinen Formel (I) gemäß den Ansprüchen 1 bis 4 auf die Unkräuter und/oder ihren Lebensraum einwirken läßt.

7. Verwendung von 4-Cyano-5-arylpyrazolen der allgemeinen Formel (I) gemäß den Ansprüchen 1 bis 4 zur Bekämpfung von Unkräutern.

8. Verfahren zur Herstellung von herbiziden Mitteln, dadurch gekennzeichnet, daß man 4-Cyano-5-arylpyrazole der allgemeinen Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 88 10 3123

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 123 420 (MAY AND BAKER LTD.) * Ansprüche 1, 2, 15-19, 21, 23, 26Q, 46, 51, 65, 67-84; Seite 3, Verbindungen 13, 17-21; Seite 5, Verbindung 78; Seite 6, Verbindungen 79-80 * | 1-8 | C 07 D 403/04<br>C 07 D 401/04<br>A 01 N 43/56 //<br>C 07 D 231/40 |
| Y | DE-A-3 426 424 (BAYER AG) * Ansprüche 1-3, 5-8; Beispiele 1, 6, 9, 12 * | 1-8 | |
| A | GB-A-2 101 999 (MAY AND BAKER LTD.) * Ansprüche 1-21, 42-73 * & DE - A - 3 226 513 (Kat. D) | 1,5-8 | |
| A | GB-A-2 136 427 (MAY AND BAKER LTD.) * Ansprüche 1, 6, 7AA, 11-19, 21-35 * | 1,4-8 | |
| A | EP-A-0 205 021 (BAYER AG) | | |

RECHERCHIERTE SACHGEBIETE (Int. Cl.4)

C 07 D 403/00
C 07 D 401/00
A 01 N 43/00

Der vorliegende Recherchenbericht wurde für alle Patentansprüche erstellt

| Recherchenort | Abschlußdatum der Recherche | Prüfer |
|---|---|---|
| BERLIN | 06-06-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)